Europäisches Patentamt

European Patent Office

Office européen des brevets

(11) Veröffentlichungsnummer: **0 029 185**
A1

# (12) EUROPÄISCHE PATENTANMELDUNG

(21) Anmeldenummer: 80106857.8

(22) Anmeldetag: 06.11.80

(51) Int. Cl.³: **A 61 M 25/00,** F 16 L 11/08

(30) Priorität: 16.11.79 DE 2946385

(43) Veröffentlichungstag der Anmeldung: **27.05.81**
**Patentblatt 81/21**

(84) Benannte Vertragsstaaten: **DE FR NL**

(71) Anmelder: **SIEMENS AKTIENGESELLSCHAFT Berlin**
**und München, Postfach 22 02 61,**
**D-8000 München 22 (DE)**

(72) Erfinder: **Gunne, Ingemar, Dr., Stationsvägen 3 B,**
**S-191 70 Sollentuna (SE)**

(54) **Kunststoffschlauch.**

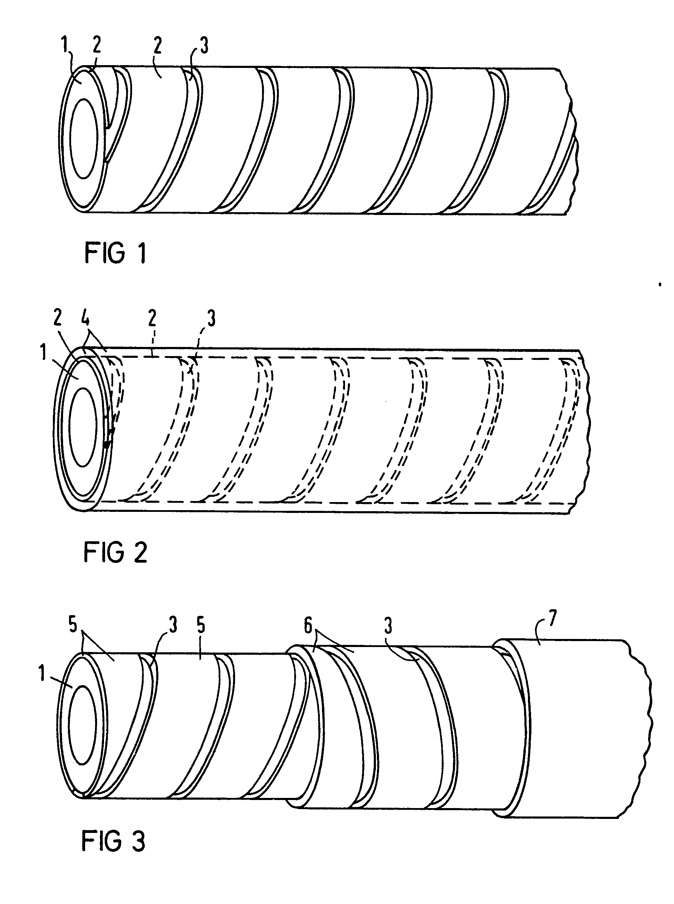

(57) Die Erfindung bezieht sich auf einen Kunststoffschlauch (1), insbesondere Katheterschlauch, der zur Erhöhung der Verwindungssteifigkeit mit einer Wendel (2)
versehen ist, die sich über seine ganze Länge erstreckt.
Die Wendel (2) besteht aus einem Kunststoff, der in der
Längsrichtung der Wendel (2) eine hohe und quer dazu
eine niedrige Zugfestigkeit besitzt (Fig. 1).

SIEMENS AKTIENGESELLSCHAFT          Unser Zeichen
Berlin und München                  VPA 79 P 5960 EUR


Kunststoffschlauch

Die Erfindung betrifft einen Kunststoffschlauch, insbesondere einen Katheterschlauch zum Einführen in einen
Patienten.

Ein Katheterschlauch aus Kunststoff für die Nierenkatheterisierung ist durch das DE-GM 18 18 183 bekannt. Dieser Nierenkatheter weist außer an seinem einführungsseitigen Ende auch in der Nähe dieses Endes und seitlich
eine Öffnung auf, durch die Kontrastmittel ausfließen
kann. Ein solcher Katheter ist verhältnismäßig lang und
elastisch, so daß er beim Einführen in einen Patienten
leicht die natürlichen Wege des zu untersuchenden Bereiches erreichen kann. Es ist bei diesem Katheter schwierig, bei eingeführtem Katheter die seitlich angeordnete
Öffnung in die gewünschte Lage zu bringen.

Der Erfindung liegt die Aufgabe zugrunde, einen Kunststoffschlauch der eingangs genannten Art zu schaffen,
der verwindungssteif ist, aber leicht gebogen werden
kann.

Lst 5 Bd / 29.10.1979

0029185

- 2 -     VPA 79 P 5960  EUR

Diese Aufgabe ist erfindungsgemäß dadurch gelöst, daß der Kunststoffschlauch zur Erhöhung der Verwindungssteifigkeit mit mindestens einer Wendel versehen ist, die sich über seine ganze Länge erstreckt und die aus einem Kunststoff besteht, der in der Längsrichtung der Wendel eine hohe und quer dazu eine niedrige Zugfestigkeit besitzt. Aufgrund der Anwendung einer Kunststoffwendel weist der Kunststoffschlauch eine hohe Verwindungssteifigkeit auf, kann aber leicht gebogen werden, wenn die Kunststoffwendel in der Querrichtung niedrige Festigkeitseigenschaften aufweist.

In einer vorteilhaften Ausbildung der Erfindung wird vorgeschlagen, daß der Kunststoffschlauch mit zwei gegenläufig zueinander laufenden Kunststoffwendeln versehen ist. Dadurch wird eine besonders hohe Verwindungssteifigkeit und dennoch eine sehr gute Biegsamkeit erzielt.

Weitere Einzelheiten der Erfindung ergeben sich aus den weiteren Unteransprüchen.

Die Erfindung ist nachfolgend anhand zweier Ausführungsbeispiele in Verbindung mit der Zeichnung näher erläutert. Es zeigen:

Fig. 1 einen Kunststoff-Katheterschlauch nach der Erfindung,

Fig. 2 eine Weiterbildung des Kunststoff-Katheterschlauches nach Fig. 1,

Fig. 3 eine weitere Ausführungsform eines Kunststoff-Katheterschlauches nach der Erfindung.

Die Fig. 1 zeigt einen Katheterschlauch 1, der aus Kunststoff besteht. Der Katheterschlauch 1 ist auf seiner Außenseite über seine ganze Länge mit einer Kunststoffwendel 2 überzogen. Die Wendel 2 besteht aus einem Kunststoffband, das ausschließlich in der Längsrichtung der
Wendel eine hohe Zugfestigkeit aufweist. Das Band kann
z.B. aus aromatischen Amid-Fasern, die in der Längsrichtung des Bandes angeordnet sind und die mit einem Polyäthylen überzogen sind, bestehen. Abhängig von der Breite und Stärke des Bandes kann die Verwindungssteifigkeit erhöht werden. In der Querrichtung des Kunststoffbandes weist der Kunststoff im Vergleich zu der Längsrichtung des Bandes niedrige Festigkeitseigenschaften
auf. Hierdurch erhält der Katheterschlauch 1 eine hohe
Verwindungssteifigkeit und gleichzeitig eine sehr gute
Biegsamkeit. Um eine noch höhere Biegsamkeit zu erhalten,
ist das Kunststoffband so gelegt, daß es einen Spalt 3
freiläßt.

In der Fig. 2 ist gezeigt, daß die Wendel 2 mit einer
Schicht 4 aus Kunststoff überzogen ist. Aufgrund dieser
gleichmäßig aufgelegten Schicht ist es sehr einfach, den
Katheter zu sterilisieren.

Die Fig. 3 zeigt, daß der Katheterschlauch 1 mit zwei gegenläufig zueinanderliegenden Wendeln 5, 6 aus Kunststoffband hoher Festigkeit in der Längsrichtung und niedriger Festigkeit in der Querrichtung, die miteinander
fest verbunden sind, versehen ist. Dies erhöht erheblich
die Verwindungssteifigkeit. Durch die niedrigen Festigkeitseigenschaften des Kunststoffbandes in der Querrichtung erhält dennoch der Katheterschlauch seine Biegsamkeit. Das Bezugszeichen 7 in dieser Figur bezieht sich
auf eine Kunststoffschicht, die auch bei diesem Ausführungsbeispiel den Katheter außen abdeckt.

Die Kunststoffwendeln 2, 5, 6 des Katheterschlauches 1
erhöhen die Festigkeit gegen Flüssigkeitsdrücke und erlauben eine verhältnismäßig dünne Wandstärke bei einer gegebenen Belastung. Die Erfindung ist nicht auf Katheterschläuche beschränkt, sondern bezieht sich auch auf Kunststoffschläuche, die auf anderen Gebieten verwendet werden, wie z.B. Schläuche für Beatmungsgeräte oder andere Schläuche, bei denen eine hohe Biegsamkeit, kombiniert mit einer hohen Verwindungssteifigkeit, erforderlich ist, z.B. Wasserschläuche.

Patentansprüche

1. Kunststoffschlauch, insbesondere Katheterschlauch, d a d u r c h   g e k e n n z e i c h n e t, daß er zur Erhöhung der Verwindungssteifigkeit mit mindestens einer Wendel (2, 5, 6) versehen ist, die sich über seine ganze Länge erstreckt und die aus einem Kunststoff besteht, der in der Längsrichtung der Wendel (2, 5, 6) eine hohe und quer dazu eine niedrige Zugfestigkeit besitzt.

2. Kunststoffschlauch nach Anspruch 1, d a d u r c h   g e k e n n z e i c h n e t, daß er mit zwei gegenläufig zueinander laufenden Kunststoffwendeln (5, 6) versehen ist.

3. Kunststoffschlauch nach Anspruch 2, d a d u r c h   g e k e n n z e i c h n e t, daß die zwei gegenläufig zueinander laufenden Kunststoffwendeln (5, 6) miteinander fest verbunden sind.

4. Kunststoffschlauch nach einem der Ansprüche 1 bis 3, d a d u r c h   g e k e n n z e i c h n e t, daß jede Kunststoffwendel (2, 5, 6) aus einem Kunststoffband besteht.

5. Kunststoffschlauch nach einem der Ansprüche 1 bis 4, d a d u r c h   g e k e n n z e i c h n e t, daß die Wendel (2) bzw. die Wendeln (5, 6) von einer Schicht (4, 7) aus Kunststoff abgedeckt sind.

FIG 1

FIG 2

FIG 3

Europäisches
Patentamt

**EUROPÄISCHER RECHERCHENBERICHT**

0029185

Nummer der Anmeldung

EP 80106857.8

| Kategorie | Kennzeichnung des Dokuments mit Angabe, soweit erforderlich, der maßgeblichen Teile | betrifft Anspruch |
|---|---|---|
| | **EINSCHLÄGIGE DOKUMENTE** | |
| X | US - A - 3 416 531 (M.L. EDWARDS)<br>+ Fig. 1,3,4; Spalte 2, Zeile 54 - Spalte 3, Zeile 20 +<br>-- | 1,2,4,5 |
| X | DE - A1 - 2 404 656 (TECHNIBIOTICS)<br>+ Seite 3, 2. Absatz - Seite 7, 1. Absatz; Fig. 2 +<br>-- | 1,5 |
| X | DE - A - 2 423 160 (OLYMPUS OPTICAL)<br>+ Fig. 4; Seite 4, letzter Absatz - Seite 5, 1. Absatz +<br>-- | 1,5 |
| X | CH - A - 596 842 (VAGN NIELS FINSEN)<br>+ Fig. 1; Spalte 1, Zeilen 46-62; Patentanspruch +<br>-- | 1,5 |
| | US - A - 3 773 034 (ITT RESEARCH INST.)<br>+ Fig. 6; Spalte 4, Zeilen 15-34 +<br>-- | 1-5 |
| | US - A - 3 428 046 (R.K. REMER et al.)<br>+ Fig. 6-9; Spalte 2, Zeilen 61-70 +<br>-- | 1 |
| A | US - A - 3 924 632 (W.A. COOK)<br>+ Gesamt +<br>---- | 1-5 |

**KLASSIFIKATION DER ANMELDUNG (Int. Cl.³)**

A 61 M 25/00
F 16 L 11/08

**RECHERCHIERTE SACHGEBIETE (Int. Cl.³)**

A 61 M 25/00
F 16 L 11/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort<br>WIEN | Abschlußdatum der Recherche<br>18-02-1981 | Prüfer<br>LUDWIG |
|---|---|---|